# EUROPEAN PATENT APPLICATION

(11) **EP 4 647 466 A1**
(43) Date of publication of application: **12.11.2025**
(21) Application number: 25173582.5
(22) Date of filing: 30.04.2025
(51) Int. Cl.: C08L 83/04, A61B 8/00

(54) **ULTRASONIC COUPLING MATERIAL COMPOSITE FILM AND UNLTRASONIC INSPECTION METHOD**

(30) Priority: 09.05.2024 JP 2024076816
(71) Applicant: Shin-Etsu Chemical Co., Ltd., Tokyo 100-0005 (JP)
(72) Inventor: Hatekayama, Jun, Niigata (JP); Ikeda, Joe, Tokyo (JP); Kobayashi, Akihiro, Gunma (JP)
(74) Representative: Schicker, Silvia

(57) **Abstract**

The present invention is an ultrasonic coupling material composite film to be inserted between an ultrasound probe surface and a skin includes a gel coupling material film made of a gel containing water, and a silicone coupling material film containing a cross-linkable silicone resin and a silicone oil having no cross-linking point. This provides: an ultrasonic coupling material composite film having contradictory properties of adhesiveness and non-adhesiveness, and an ultrasonic inspection method using the composite film.

## Description

### TECHNICAL FIELD

The present invention relates to: an ultrasonic coupling material composite film to be brought into contact with the skin of a living body and used to visualize the state of organs and blood vessels in the body and measure pulse rate and blood pressure; and an ultrasonic inspection method.

### BACKGROUND ART

In recent years, wearable devices have been developed with the spread of Internet of Things (IoT). Watches and glasses that can connect to the Internet are typical examples. In the medical and sports fields, wearable devices that enable constant monitoring of the body's condition have been required and are growing sectors in the future.

For example, a watch-type wearable device equipped with an optical sensor, a capacitance sensor, an ultrasonic sensor, a pressure-sensitive sensor, or the like has been proposed (Patent Document 1).

Wearable devices that enable ultrasonic diagnosis (inspection) have been studied. In this case, it is possible to visualize the state of organs and blood vessels in the body and to measure pulse rate and blood pressure at all times (Patent Document 2).

In an ultrasound diagnostic instrument, an ultrasound probe is placed in close contact with the skin, ultrasound waves emitted from the probe surface through an ultrasound transmitter are reflected inside the body, and information inside the body can be detected by a receiver through the probe. During ultrasonic measurement, if a surface to be measured is uneven and the surface of the ultrasound probe fails to cover the unevenness of the surface to be measured, resulting in a layer of air, ultrasound waves emitted from the surface of the ultrasound probe are reflected by the layer of air and do not reach the inside of the body. The ultrasound transmission speed in a silicone rubber ultrasound probe at room temperature is approximately 1000 m/s, while the ultrasound speed in air is 340 m/s. Because of the large difference in speed, ultrasound transmission is hindered at the interface between the ultrasound probe and air. Thus, it is necessary to prevent the presence of air between the ultrasound probe and the skin. Therefore, a water-soluble gel-based coupling material is inserted between the surface of the ultrasound probe and the skin in ultrasonic diagnosis (Patent Document 3). Patent Document 4 provides many examples of applications of water-soluble coupling materials.

For a one-time ultrasonic measurement, a cream of water-soluble gel-based coupling material is applied to the skin, or a film of water-soluble gel-based coupling material is placed in close with the skin, and an ultrasound probe is placed in close contact thereon to perform measurement. After the measurement, the cream coupling material is wiped off with cloth or paper or washed off with water. However, if the users have the ultrasound probe affixed to them for a long time and do exercise with sweating or take a shower or bath with the ultrasound probe applied, the cream water-soluble gel dissolves. Without the coupling material, an ultrasound signal is no longer received. The cream gel coupling material therefore cannot be used in the applications of long-term attachment.

For this reason, non-water soluble coupling materials have been proposed. For example, a paraffin-based material (Patent Document 5) and a silicone gel-based material have been proposed (Patent Document 6).

Gels containing water are superior in terms of a low attenuation rate and a high transmission speed of ultrasound. A water-containing ultrasonic coupling material with hydrophobic urethane as a gel has been proposed to prevent drying (Patent Document 7).

A laminated coupling material has been proposed in which a water-gel layer is covered with a membrane to prevent drying, and an adhesive layer is further provided on the outside (Patent Document 8). This coupling material is provided between an ultrasound probe and the skin to enable long-term continuous diagnosis with a sticker-type ultrasound sensor.

Before ultrasonic diagnosis, an ultrasonic diagnosis site needs to be identified. For example, in diagnosing the movement of the carotid arteries in the neck, an ultrasound probe with an ultrasonic coupling material is traced over the skin of the neck or repeatedly applied and uncoupled to find the optimal location where the carotid arteries can be observed. In this case, the ultrasonic coupling material does not need to be adhesive, and it is preferable that it have high releasability.

Thus, the ultrasonic coupling material requires contradictory properties of adhesiveness and non-adhesiveness, and materials having these properties have been sought for.

### CITATION LIST

### PATENT LITERATURE

Patent Document 1: JP 2021-093127 A
Patent Document 2: WO 2020/049934 A1
Patent Document 3: JP S46-000146 A
Patent Document 4: JP 2005-532871 A
Patent Document 5: JP S63-019135 A
Patent Document 6: US 9211106 B2
Patent Document 7: JP 2020-183929 A
Patent Document 8: US 2023/0277159 A1

### SUMMARY OF INVENTION

### TECHNICAL PROBLEM

The present invention has been made in order to solve the above problem and aims to provide an ultrasonic coupling material composite film having contradictory properties of adhesiveness and non-adhesiveness, and an ultrasonic inspection method using the composite film.

### SOLUTION TO PROBLEM

To solve the above problem, the present invention provides an ultrasonic coupling material composite film to be inserted between an ultrasound probe surface and a skin, the ultrasonic coupling material composite film comprising: a gel coupling material film comprising a gel containing water; and a silicone coupling material film comprising a cross-linkable silicone resin and a silicone oil having no cross-linking point.

The ultrasonic coupling material composite film according to the present invention can balance contradictory properties of adhesiveness and non-adhesiveness.

In the composite film, it is preferable that the gel coupling material film have adhesiveness.

With such a composite film, long-term ultrasonic inspection can be performed while an ultrasound probe with the adhesive gel coupling material film is affixed to the skin at a point to be inspected.

In the present invention, it is preferable that the silicone coupling material film comprise a cured product of an addition-curable silicone coupling material composition comprising a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst.

By using such an addition-curable silicone coupling material composition, an ultrasonic coupling material composite film having a non-adhesive (low adhesive) silicone coupling material film can be obtained.

In this case, a number of moles of SiH groups of the component (C) divided by a number of moles of alkenyl groups of the component (A) is preferably in a range of 0.5 to 20.

It is also preferable that the component (B) be a linear or branched diorganopolysiloxane which has no cross-linking point, not have an alkenyl group and a SiH group, and have a kinematic viscosity in a range of 10 to 50000 mm²/s at 25°C.

In the present invention, it is preferable that a mass of the component (B) divided by a total mass of the component (A), the component (B), and the component (C) be in a range of 0.20 to 0.90.

By using such an addition-curable silicone coupling material composition, the non-adhesiveness of the silicone coupling material film can be adjusted as appropriate.

In the present invention, it is preferable that the addition-curable silicone coupling material composition further comprise 0.1 to 10 parts by mass of dry silica per 100 parts by mass of a total of the components (A) to (C).

This configuration can effectively prevent bleed-out of silicone oil.

In the present invention, it is preferable that the silicone coupling material film have a thickness in a range of 10 to 5000 µm.

In the present invention, the silicone coupling material film having such a thickness can be used.

In the present invention, it is preferable that the ultrasonic coupling material composite film further comprise membranes on both sides of the gel coupling material film and adhesive films outside the membranes.

The membranes on both sides of the gel coupling film can prevent the water gel from drying.

In the present invention, it is preferable that the ultrasonic coupling material composite film further comprise a release liner film on one side or both sides of the ultrasonic coupling material composite film.

By using such a release liner film, the ultrasonic coupling material composite film of the present invention can be efficiently obtained.

The present invention also provides an ultrasonic inspection method using the above ultrasonic coupling material composite film, excluding methods for operating on, treating, or diagnosing humans, the method comprising:
inserting the ultrasonic coupling material composite film between an ultrasound probe surface and a subject such that the gel coupling material film of the composite film faces the ultrasound probe surface and the silicone coupling material film of the composite film faces the subject; and
bringing the silicone coupling material film into contact with the subject to search for an ultrasonic inspection point.

In such an ultrasonic inspection method, the ultrasonic coupling material composite film with contradictory properties of adhesiveness and non-adhesiveness is used, so that the non-adhesive (low adhesive) silicone coupling material (film) enables sliding on the skin or repeated attachment and uncoupling on the skin to search for an optimal ultrasonic inspection point, and the gel, for example, adhesive gel, coupling material (film) enables affixing to the skin for a long time. With such an ultrasonic inspection method using the ultrasonic coupling material composite film, the optimal ultrasonic inspection point can be searched for by repeating contact with the skin and uncoupling from the skin or by sliding aside on the skin, and once the ultrasonic inspection point is found, the gel coupling material film can be affixed to the skin after stripping off the non-adhesive coupling material film.

In the ultrasonic inspection method according to the present invention, the ultrasonic inspection point can be searched for by repeating contact with the subject and uncoupling from the subject or by sliding and moving on the subject. In addition, continuous ultrasonic inspection can be performed by uncoupling the silicone coupling material film and then immobilizing the gel coupling material film in contact with the subject.

In this way, with the use of the ultrasonic coupling material composite film according to the present invention, the optimal ultrasonic inspection point can be searched for by repeating contact with the skin and uncoupling from the skin or by sliding aside on the skin, and once the ultrasonic inspection point is found, the gel, for example, adhesive gel, coupling material film can be affixed to the skin by stripping the non-adhesive silicone coupling material film. In this way, ultrasonic inspection can be performed efficiently.

The present invention also provides a method for forming a layered structure. The method includes: applying, on a release liner film, an addition-curable silicone coupling material composition containing a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst, and heating and curing the addition-curable silicone coupling material composition to form a silicone coupling material film; then affixing the silicone coupling material film on a gel coupling material film made of a gel containing water; and attaching an ultrasound probe on a surface of the gel coupling material film opposite a surface in contact with the silicone coupling material film to form a layered structure.

Further, a layered structure can be formed by affixing the gel coupling material film to the ultrasound probe by stripping the silicone coupling material film from the gel coupling material film.

In this way, by forming the layered structure appropriately, ultrasonic inspection can be performed efficiently.

### ADVANTAGEOUS EFFECTS OF INVENTION

As described above, the ultrasonic coupling material composite film according to the present invention can balance contradictory properties of adhesiveness and non-adhesiveness. For example, when the non-adhesive silicone coupling material (film) is brought into contact with a subject such as skin, an optimal ultrasonic inspection point can be searched for by sliding on the skin or repeating attaching and uncoupling on the skin. When the gel, for example, adhesive gel, coupling material (film) is brought into contact with the subject, affixing to the skin for a long time is possible, enabling stable ultrasonic inspection. With the ultrasonic inspection method using such an ultrasonic coupling material composite film, an optimal ultrasonic inspection point can be searched for by repeating contact with the skin and uncoupling from the skin or by sliding aside on the skin, and once the ultrasonic inspection point is found, the gel, for example, adhesive gel, coupling material film can be affixed to the skin by stripping the non-adhesive silicone coupling material film.

### BRIEF DESCRIPTION OF DRAWINGS

FIG. 1 is a schematic cross-sectional view of a releasable silicone coupling material film according to an embodiment of the present invention;
FIG. 2 is a schematic cross-sectional view of a composite film of the releasable silicone coupling material film and a water-containing gel coupling material film (water-gel coupling material film) according to an embodiment of the present invention;
FIG. 3 is a schematic cross-sectional view of an ultrasonic coupling material composite film applied on an ultrasound probe according to an embodiment of the present invention;
FIG. 4 is a schematic cross-sectional view of the ultrasound probe with the ultrasonic coupling material composite film applied to the skin according to an embodiment of the present invention;
FIG. 5 is a schematic cross-sectional view of the ultrasound probe with the adhesive water-containing coupling material film affixed to the skin after a diagnostic site is identified and the releasable silicone coupling material film is uncoupled off, according to an embodiment;
FIG. 6 is a schematic cross-sectional view of a configuration in which the water-containing coupling material film in FIG. 4 has membranes on both sides of the water-gel coupling material film and adhesive films outside the membranes, according to an embodiment;
FIG. 7 is a schematic cross-sectional view in which the water-gel coupling material film exposed by stripping the silicone coupling material film from the coupling material composite film in FIG. 6 is applied to the skin, according to an embodiment;
FIG. 8 is a cross-sectional view of the strippable silicone coupling material film being stripped off;
FIG. 9 is a cross-sectional view of the strippable silicone coupling material film being stripped off using a stick with a circular cross section;
FIG. 10 is a cross-sectional view of ultrasound waves emitted from the ultrasound probe and transmitted to the skin through the coupling material;
FIG. 11 is a cross-sectional view of ultrasound waves emitted from the ultrasound probe and not transmitted to the skin through the coupling material;
FIG. 12 is a photograph of an ultrasound diagnostic instrument as viewed from the ultrasound probe;
FIG. 13 is a photograph of ultrasonic diagnosis being performed on a carotid artery area of the neck;
FIG. 14 is a photograph of an ultrasonogram of the carotid artery area when the ultrasonic coupling material composite film according to the present invention is used; and
FIG. 15 is a photograph of an ultrasonogram of the carotid artery area when a layered ultrasonic coupling material according to a comparative example is used.

### DESCRIPTION OF EMBODIMENTS

As described above, there is a need for both a non-adhesive ultrasonic coupling material to be slid on the skin or repeatedly attached to and uncoupled from the skin to search for an optimal ultrasonic inspection point, and an adhesive ultrasonic coupling material that can be affixed to the skin for a long time.

The inventors of the present invention have conducted elaborate studies on the above problem and conceived an ultrasonic coupling material composite film with a layered structure having a non-adhesive silicone coupling material film on the skin side of a gel coupling material film (which may be adhesive). The inventors have found that such a composite film can be used to search for an ultrasonic inspection point by repeating contact with the skin and uncoupling from the skin or by sliding horizontally on the skin, and once the ultrasonic inspection point is found, the gel coupling material film can be affixed to the skin by stripping the non-adhesive silicone coupling material film. This finding has led to completion of the invention. In the following, ultrasonic diagnosis may be described as a mode of ultrasonic inspection.

The non-adhesive silicone coupling material film may be a silicone-based coupling material film. This is because silicone has excellent stripping properties and can be easily stripped off when layered with an adhesive gel coupling film.

Specifically, the present invention provides an ultrasonic coupling material composite film to be inserted between an ultrasound probe surface and a skin. The ultrasonic coupling material composite film includes a gel coupling material film made of a gel containing water, and a silicone coupling material film containing a cross-linkable silicone resin and a silicone oil having no cross-linking point.

Patent Document 6 described above discloses a coupling material using a silicone gel. The silicone gel is a very soft silicone rubber with ultra-low-density cross-linking. However, softness alone is not sufficient to fill minute roughness of soft skin on the order of micron.

To further soften the silicone gel, non-cross-linkable silicone oil or hydrocarbon-based mineral oil may be added, as described in JP 2015-028178 A. The addition of non-cross-linkable silicone oil improves the skin roughness filling properties and improves the sensitivity of ultrasonic measurement when used as an ultrasonic coupling material. However, if a large amount of non-cross-linkable silicone oil is added, the silicone oil bleeds out, causing silicone oil residues on the skin when the coupling film is uncoupled from the skin after ultrasonic measurement is finished. The silicone oil residues are undesirable because it is necessary to wipe the skin with a towel or cotton ball soaked in mineral oil to remove the residues, which is time-consuming.

Adding dry silica is effective in preventing bleed-out of silicone oil. Adding an MQ resin is also effective in preventing bleed-out of silicone oil but is not preferable for this application because adhesiveness is developed. It is preferable to add dry silica to prevent bleed-out of silicone oil without involving adhesiveness.

The ultrasound attenuation rate in water is small, almost the same as in the human body, and the transmission speed in water is also the same as in the human body. A water-containing gel has excellent ultrasound transmission properties. On the other hand, the ultrasound attenuation rate in silicone is lower than in urethane or acrylic, but higher than in water. If the ultrasound attenuation rate is high, ultrasonograms obtained with a coupling material including this are dark and have lower contrast. Using a water-gel coupling material is advantageous in precise ultrasonic measurement. However, finding an ultrasonic measurement point does not require ultrasound transmission properties as high as those of water gel, but rather can take advantage of the silicone coupling material with high uncoupling properties.

The water-gel ultrasonic coupling material is a composition of a cross-linkable water-soluble polymer containing water, and the compositions described in Patent Documents 3, 4, and 7 can be used.

The present invention will be described in detail below. However, the present invention is not limited thereto.

### <Silicone Coupling Material Composition>

The silicone coupling material (silicone ultrasonic coupling material) composition used in the ultrasonic inspection method according to the present invention contains a cross-linkable silicone rubber and a silicone oil having no cross-linking points. Each component will be described in detail below.

The silicone coupling material film composition to be used in the ultrasonic inspection method according to the present invention is preferably an addition-curable silicone coupling material composition containing a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst.

### Component (A)

The component (A) blended in the silicone coupling material composition according to the present invention is a diorganopolysiloxane having an alkenyl group. For example, the diorganopolysiloxane having an alkenyl group may be a diorganopolysiloxane which corresponds to component (A1) described in JP 2015-193803 A and has a viscosity of 100,000 mPa·s or more at 25°C.

Examples of the alkenyl group include vinyl, allyl, hexenyl, octenyl, acryloylpropyl, acryloylmethyl, methacryloylpropyl, and vinyloxypropyl groups. In particular, a vinyl group is preferred.

A group other than the alkenyl group is a substituted or unsubstituted monovalent hydrocarbon group having 1 to 10 carbon atoms. Specific examples include alkyl groups such as methyl, ethyl, propyl, and butyl groups, cycloalkyl groups such as cyclohexyl group, aryl groups such as phenyl and tolyl groups, and those in which some or all of hydrogen atoms bonded to carbon atoms of these groups are replaced with other groups such as halogens, amino groups, hydroxyl groups, or cyano groups, such as 3-aminopropyl, 3,3,3-trifluoropropyl, 3-hydroxypropyl, and 3-cyanopropyl groups. In particular, methyl and phenyl groups are preferred.

Furthermore, a diorganopolysiloxane (A2) which corresponds to component (A2) described in JP 2015-193803 A, has a viscosity of 100,000 mPa·s or more at 25°C, and has no alkenyl group may be contained as the component (A). The polysiloxanes (A1) and (A2) above may be linear or branched.

Silicone-based resins include addition reaction curable-type resins and radical cross-linking reaction curable-type resins. In the addition-curable type, an alkenyl group and a SiH group are cross-linked via an addition reaction, whereas in the radical-curable type, cross-linking proceeds not only in the presence of an alkenyl group but also in the absence of an alkenyl group. In this case, cross-linking with the silicone oil component (B) having no cross-linking group also may occur. This may reduce the fluidity of the silicone oil and reduce skin roughness filling performance to decrease sensitivity as an ultrasonic coupling material. For this reason, the addition-curable type is preferred compared with the radical cross-linking type.

A modified siloxane having a group selected from the following can be added as the component (A): amino, oxirane, oxetane, polyether, hydroxy, carboxyl, mercapto, methacryl, acryl, phenol, silanol, carboxylic anhydride, aryl, aralkyl, amide, and ester groups, and lactone ring. The modified siloxane may be a siloxane in which one end, both ends, or a side chain is modified.

It is effective to reduce the amount of cyclic siloxane such as octamethylcyclotetrasiloxane and decamethylcyclopentasiloxane in the component (A) in order to reduce skin irritation. A method for reducing the amount of cyclic siloxane is described in JP 2015-193803 A.

The components (A1) and (A2) may be in an oil form or a raw rubber form. In the case of the raw rubber form, if the viscosity is as high as more than 500,000 mPa·s, the viscosity of a solution when the raw rubber is dissolved in toluene to a concentration of 30% by mass (30% dissolution viscosity) is preferably 100,000 mPa·s or less. Furthermore, the 30% dissolution viscosity is preferably 1,000 to 60,000 mPa·s. If the 30% dissolution viscosity is 100,000 mPa·s or less, the composition does not become highly viscous and does not become difficult to agitate during production. In the present invention, the viscosity (absolute viscosity) is a value determined with a rotary viscometer at 25°C.

### Component (B)

The component (B) is a silicone oil having no cross-linking point, preferably a linear or branched diorganopolysiloxane which has no a cross-linking point, does not have an alkenyl group and a SiH group, and has a kinematic viscosity in a range of 10 to 50000 mm²/s at 25°C. The kinematic viscosity at 25°C is more preferably in a range of 50 to 50000 mm²/s. The term "cross-linking point" refers to a point of cross-linking between molecules such as alkenyl group and SiH group.

For example, as such a non-cross-linkable silicone oil, KF-96 available from Shin-Etsu Chemical Co., Ltd. can be used. The kinematic viscosity at 25°C corresponds to the number immediately before CS in the product name. For example, the kinematic viscosity of "KF-96 1,000CS" is 1,000 mm²/s and the kinematic viscosity of "KF-96 100CS" is 100 mm²/s. The kinematic viscosity is a value measured with an Ostwald viscometer at 25°C.

The component (B) is a fluid oil by itself at room temperature. The addition of the component (B) can improve the ultrasound transmission properties by filling minute roughness of the skin when the ultrasonic coupling film is affixed to the skin.

The mass of the component (B) divided by the total mass of the component (A), the component (B), and the component (C) is preferably in a range of 0.20 to 0.90, more preferably in a range of 0.30 to 0.90, and even more preferably in a range of 0.30 to 0.88.

### Component (C)

The component (C) is an organohydrogenpolysiloxane having a SiH group, which corresponds to component (C) described in JP 2015-193803 A. The component (C) preferably has a viscosity at 25°C of 1 to 1,000 mPa·s, and even more preferably 2 to 500 mPa·s. The component (C) may be a mixture of two or more types. The component (C) may be a linear, branched or cyclic structure. The number of SiH groups is preferably two or more per molecule of the organohydrogenpolysiloxane.

The diorganosiloxane (A1) having an alkenyl group and the organohydrogenpolysiloxane (C) having a plurality of SiH groups can be cross-linked via an addition reaction using a platinum catalyst. The ratio of the number of moles of SiH groups divided by the number of moles of alkenyl groups is preferably in a range of 0.5 to 20 and more preferably in a range of 1 to 15.

### Component (D)

Examples of the component (D) which is platinum group metal catalyst include platinum-based catalysts such as chloroplatinic acid, alcohol solutions of chloroplatinic acid, reaction products of chloroplatinic acid and alcohols, reaction products of chloroplatinic acid and olefin compounds, reaction products of chloroplatinic acid and vinyl group-containing siloxanes, platinum-olefin complexes, and platinum-vinyl group-containing siloxane complexes, rhodium-based catalysts such as rhodium complexes, and ruthenium-based catalysts such as ruthenium complexes. A solution or dispersion of these catalysts in an alcohol solvent, a hydrocarbon solvent, or a siloxane solvent may also be used.

The amount of the platinum group metal catalyst added is preferably in a range of 5 to 2,000 ppm (in terms of metal mass), particularly in a range of 10 to 500 ppm, per 100 parts by mass of a resin solution combining the (A), (B) and (C) and the organic solvent.

When the addition-curable silicone resin is used, an addition reaction control agent may be added. The addition reaction control agent is added as a quencher for preventing the platinum group metal catalyst from acting in the solution and in a low-temperature environment before heat curing after film formation. Specific examples include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, and 1,1,3,3-tetramethyl-1,3-divinyldisiloxane.

The amount of the addition reaction control agent added is preferably in a range of 0 to 10 parts by mass, particularly in a range of 0.05 to 3 parts by mass, per 100 parts by mass of the resin solution.

### Silica Particles

Silica particles can be blended into the silicone coupling material composition according to the present invention. In particular, dry silica particles can be added. The addition of dry silica improves the strength of the ultrasonic coupling material film, makes the film less likely to tear when removed from the release liner, facilitates application on skin or an ultrasound probe, prevents bleed-out of silicone oil of the component (B), and prevents the silicone oil from sticking to the skin after uncoupling from the skin and reducing the adhesiveness when the water-gel coupling material film is subsequently affixed. The dry silica has a specific surface area in a range of 50 to 500 m²/g and preferably 100 to 400 m²/g. The specific surface area can be measured by the BET method. Silica in which a silanol on the silica surface is substituted with a trimethylsilyl group or a dimethylsilyl group or dimethylsilicone is preferred because of its high dispersibility.

The dry silica may be a commercially available product. For example, AEROSIL RX200 (available from Nippon Aerosil Co., Ltd.) can be used.

The amount of silica added is preferably 0.1 to 10 parts by mass of dry silica per 100 parts by mass of a total of the components (A) to (C).

### Organic Solvent

An organic solvent can be added to the composition of the silicone coupling material according to the present invention. Specific examples of the organic solvent include aromatic hydrocarbon solvents, such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; and aliphatic hydrocarbon solvents, such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, n-tridecane, n-pentadecane, n-hexadecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin.

The amount of the organic solvent added is preferably in a range of 10 to 50,000 parts by mass per 100 parts by mass of the total of the components (A) and (B) .

It is preferable that the component (A) should not contain an MQ resin which develops adhesiveness. The MQ resin contains a solvent because it can be stable in a non-polar solvent. In the absence of the MQ resin containing a solvent, the composition of the silicone coupling material to be used in the ultrasonic inspection according to the present invention does not necessarily contain an organic solvent.

### Other Additives

In addition to the above dry silica, inorganic particles and pigments such as wet silica, alumina particles, titania particles, and zirconia particles can also be mixed in the silicone coupling material composition according to the present invention. The addition of wet silica particles, alumina particles, titania particles, or zirconia particles improves strength. For coloring, a pigment is added. The alumina particles, titania particles, and zirconia particles can preferably be used in either dry or wet, and treating their surfaces with a water-repellent silylating agent provides excellent dispersibility. The shape of the wet silica, alumina particles, titania particles, and zirconia particles can be spherical, elliptical, irregular-shaped, hollow, or porous.

### <Ultrasonic Coupling Material Composite Film>

The ultrasonic coupling material composite film according to the present invention includes a gel coupling material film made of a gel containing water, and a silicone coupling material film containing a cross-linkable silicone resin and a silicone oil having no cross-linking point, and is inserted between an ultrasound probe surface and a skin.

The composite film according to the present invention can include both the adhesive gel coupling material film and the non-adhesive silicone coupling material film and thereby can achieve contradictory adhesiveness.

The ultrasonic coupling material composite film according to the present invention will be described in detail below with reference to the drawings. However, the present invention is not limited thereto.

### <Silicone Coupling Material Film>

In the present invention, a silicone coupling material film containing a cross-linkable silicone resin and a silicone oil having no cross-linking point is used as the silicone coupling material film. This silicone coupling material film is non-adhesive and can be disposed on the water-gel coupling film, which may have adhesiveness, to be inserted between a subject such as skin and the water-gel coupling film, so that ultrasound waves can be transmitted between the skin and the ultrasound probe through the silicone coupling material film without direct contact of the water-gel coupling film with the skin.

The silicone coupling material film can be formed using the silicone coupling material composition on a release liner film.

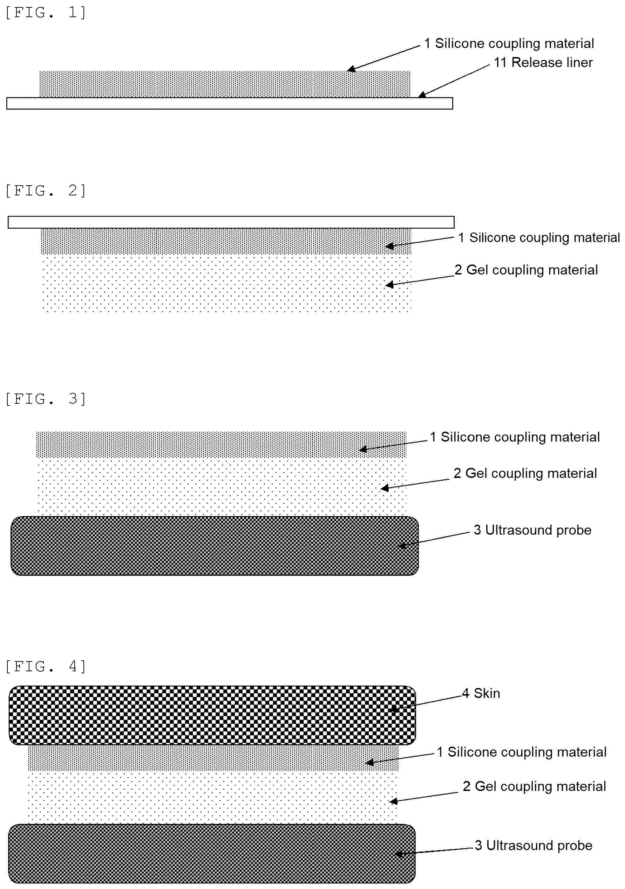

FIG. 1 is a schematic cross-sectional view of a silicone coupling material film according to an embodiment of the present invention. FIG. 1 illustrates a silicone coupling material 1 formed on a release liner 11.

A base material for the release liner can be selected from paper, plastic films, glass, metal, cloth, and the like. Examples of the paper include fine paper, coated paper, art paper, glassine paper, polyethylene laminated paper, kraft paper, Japanese paper, and synthetic paper. Examples of the plastic films include polyethylene film, polypropylene film, polyester film, polyimide film, polyamide film, polyvinyl chloride film, polyvinylidene chloride film, polyvinyl alcohol film, polycarbonate film, polytetrafluoroethylene film, polystyrene film, ethylene-vinyl acetate copolymer film, ethylene-vinyl alcohol copolymer film, triacetyl cellulose film, polyether ether ketone film, and polyphenylene sulfide film. The glass is not particularly limited in thickness, type, and the like. Chemically strengthened glass may be used. Glass fibers may be employed. The glass fibers may be used alone or in combination with other resins. Examples of the cloth include natural fiber cloth, synthetic fiber cloth, and artificial leather. Examples of the metal include aluminum foil, copper foil, gold foil, silver foil, and nickel foil. Among these, paper and plastic films are preferred, and in particular, a polyester film is preferred.

A fluorinated release agent described in JP 2020-100763 A or JP 2020-100764 A may be applied on the base material of the release liner to reduce release force.

### <Water-Gel Coupling Material Film>

The water-gel coupling material film that can be used in the present invention may be any gel coupling material film that is made of a gel containing water, which is not limited specifically, but preferably has adhesiveness. The water-containing gel is excellent in terms of a low attenuation rate and a high transmission speed of ultrasound.

A water-soluble gel subjected to water-resistant treatment or the like as necessary can be used as the water-gel coupling material (film). However, the water-soluble gel may be removed by sweat or the like, so it is preferable to use a non-water-soluble gel coupling material. The non-water-soluble gel coupling material is not limited. Examples include an ultrasonic coupling material containing water with hydrophobic urethane as a gel as described in Patent Document 7, and a laminated coupling material having a water-gel layer covered with a membrane and an adhesive layer provided on the outside as described in Patent Document 8. The gel coupling material is arranged between an ultrasound probe and a skin to enable long-term continuous diagnosis with a sticker-type ultrasound sensor. An adhesive such as acrylic adhesive may be applied on both sides of the water-gel coupling material film to form an adhesive water-gel coupling material film.

A commercially available product may be used as the water-gel coupling material film. For example, a water-containing urethane gel such as Yasojima Echo Gel Pad EP-S-03 (available from Yasojima Proceed Co., Ltd.) can be used.

### <Method for Producing Ultrasonic Coupling Material Composite Film>

The ultrasonic coupling material composite film according to the present invention can be produced as follows. However, the production method is not limited to the following as long as the composite film of the present invention can be obtained.

### <Method for Producing Silicone Coupling Material Film>

As illustrated in FIG. 1, a silicone coupling material composition is applied on the release liner 11 to form the silicone coupling material film 1 on the release liner 11. A method of applying the ultrasonic coupling material composition on the base material of the release liner is not limited. Preferred examples include dip coating, spray coating, spin coating, bar coating, comma coating, slit coating, roll coating, flow coating, doctor coating, screen printing, flexographic printing, gravure printing, and inkjet printing. The composition described above is preferably used as the silicone coupling material composition.

A method of curing the silicone coupling material composition is not limited. For example, it is preferable that the applied film be cured by either heat or light, or both.

The temperature for heating is not limited and for example, preferably 50 to 250°C. Examples of the heating method include electric heating such as hot air and hot plate, and heat generation such as infrared and microwave irradiation.

When the heating is combined with light irradiation or infrared or microwave irradiation, the heating and the light irradiation or infrared or microwave irradiation may be performed simultaneously, or the heating may be performed after the light irradiation or infrared or microwave irradiation, or the light irradiation or infrared or microwave irradiation may be performed after the heating. Air drying may be performed for the purpose of evaporating the solvent before heating after film application.

The silicone coupling material film heated and cured preferably has a thickness in a range of 10 to 5000 µm, more preferably 10 to 1000 µm, and even more preferably 20 to 800 µm.

The silicone coupling material composition may be applied on the water-gel ultrasonic coupling material film (water-gel coupling material film), rather than applied on the release liner.

The silicone coupling material film may be sandwiched between release liners. This structure facilitates transport of the soft silicone coupling material film.

As illustrated in FIG. 2, the silicone coupling material film 1 is directly affixed onto the water-gel, for example, adhesive water-gel, coupling material film 2. When the silicone coupling material film is sandwiched between release liners on both sides, the release liner on one side is removed, and the silicone coupling material film 1 on the side from which the release liner is removed is affixed onto the water-gel coupling material film 2.

As illustrated in FIG. 3, a composite film of the silicone coupling material film 1 and the water-gel coupling material 2 can also be affixed onto the ultrasound probe 3.

As illustrated in FIG. 4, the ultrasound probe 3 with the composite film of the silicone coupling material film 1 and the water-gel coupling material 2 is repeatedly applied to and uncoupled from the skin 4 or displaced horizontally to search for an optimal diagnosis point.

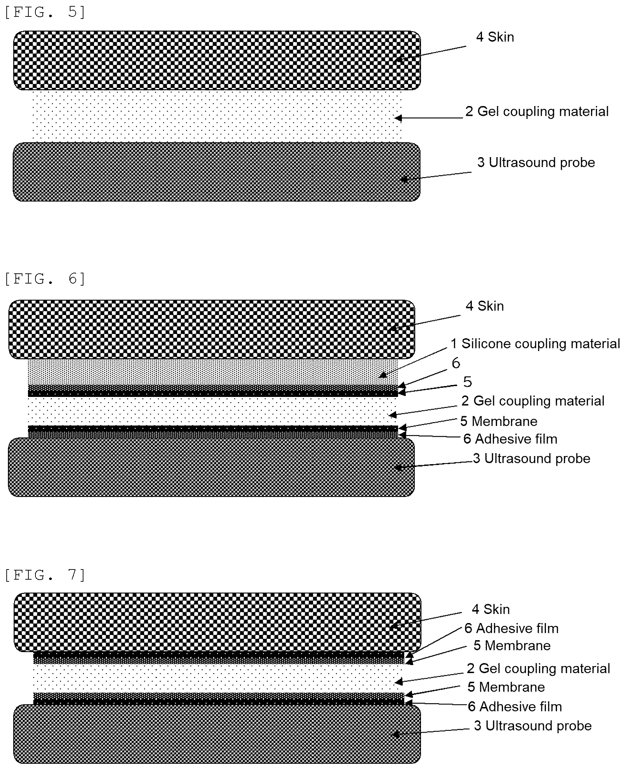

Once the diagnosis point is found, the silicone coupling material 1 is stripped and, as illustrated in FIG. 5, the ultrasound probe with the water-gel coupling material film is affixed to the skin to perform ultrasonic diagnosis for a long time.

FIG. 6 illustrates a composite film including the water-gel coupling material film 2 with membranes 5 and adhesive films 6 on both sides as disclosed in Patent Document 8. The membranes are for the purpose of preventing drying of the water gel and are not always necessary for adhesion to the skin.

FIG. 7 illustrates the composite film (composite ultrasonic coupling material) affixed to the skin 4, with the silicone coupling material film 1 in FIG. 6 stripped off.

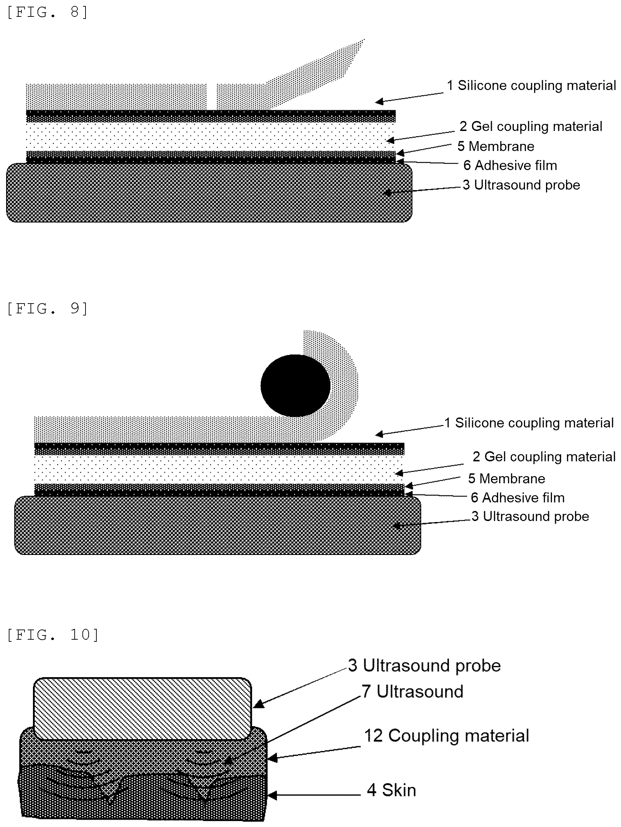

FIG. 8 illustrates the silicone coupling material film 1 being stripped off.

FIG. 9 illustrates the silicone coupling material film 1 being stripped off while being wound around a stick.

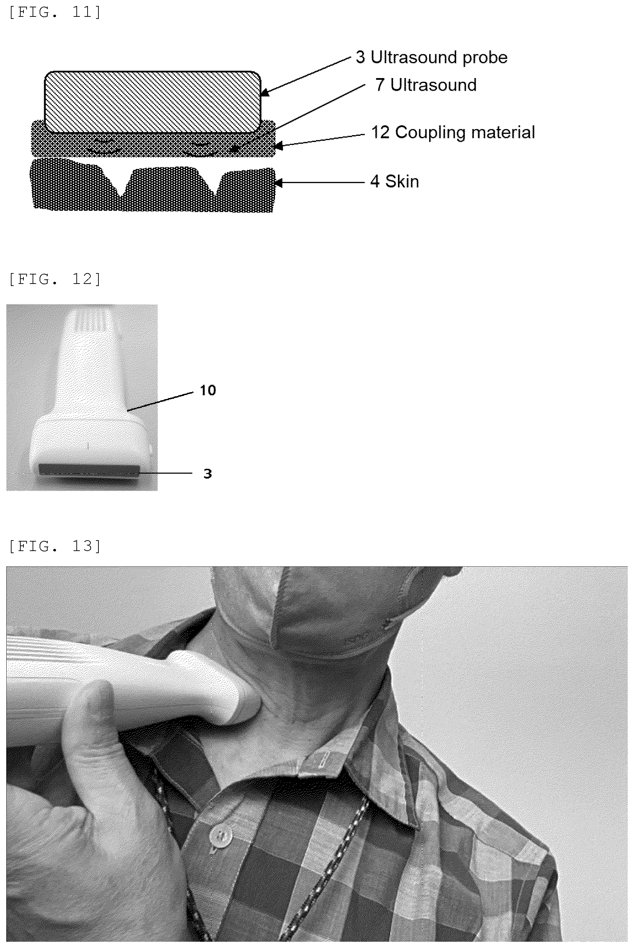

Ultrasound waves emitted from an ultrasound probe and transmitted into the skin through the coupling material in ultrasonic diagnosis will be described with reference to FIGs. 10 and 11. Ultrasound here is generally a sound with a frequency of 3 MHz or higher for use in ultrasonic diagnosis.

FIG. 10 is a schematic cross-sectional view of a coupling material 12 sandwiched between the skin 4 and the ultrasound probe 3. Since the coupling material 12 fills the roughness of the skin 4, ultrasound waves 7 emitted from the ultrasound probe 3 are transmitted into the skin.

On the other hand, FIG. 11 is also a schematic cross-sectional view of the coupling material 12 sandwiched between the skin 4 and the ultrasound probe 3. In this case, however, the coupling material 12 does not fill the roughness of the skin 4, so the ultrasound waves 7 are not transmitted into the skin.

As described above, because of the high ultrasound transmission speed in silicone rubber relative to air, ultrasound transmission is hindered at the interface between the ultrasound probe and the air. Therefore, if an air layer exists between the surface to be measured and the ultrasound probe surface during ultrasonic measurement, the ultrasound waves emitted by the ultrasound probe are reflected by the air layer and do not reach the inside of the body (FIG. 11). Thus, in order to prevent the presence of the air between the ultrasound probe and the skin, a water-soluble gel-based coupling material or the like is inserted between the ultrasound probe surface and the skin during ultrasonic diagnosis (FIG. 10).

In the present invention, the ultrasonic coupling material composite film having a layered structure including the non-adhesive silicone coupling material film on the skin side of the gel coupling material film (which may be adhesive) is used, so that (1) when the presence of air between the ultrasound probe 3 and the skin 4 is permitted, that is, while an optimal ultrasonic inspection point is searched for by repeating contact with the skin or uncoupling from the skin or by sliding aside on the skin, the non-adhesive silicone coupling material film 1 can be sandwiched between the skin 4 and the gel coupling material film 2 (FIG. 4), and (2) when it is necessary to avoid the presence of air between the ultrasound probe and the skin, that is, after the ultrasonic inspection point is identified, the gel coupling material film 2 can be affixed directly to the skin 4 by stripping the non-adhesive silicone coupling material film 1 (FIG. 5). In this way, the present invention achieves speedy identification of an ultrasonic inspection point and a suitable inspection result (clear ultrasonic inspection image) at the same time.

The silicone coupling material film containing a cross-linkable silicone resin and a silicone oil having no cross-linking point is hydrophobic and functions to prevent drying of the gel coupling material containing water. The hydrophobic silicone oil can prevent mixing with water.

FIG. 12 is a photograph of a hand-held ultrasound diagnostic instrument 10 as viewed from the ultrasound probe 3.

FIG. 13 is a photograph in which an ultrasonogram of a carotid artery area is measured with the coupling material composite film attached on the probe of the hand-held ultrasound diagnostic instrument.

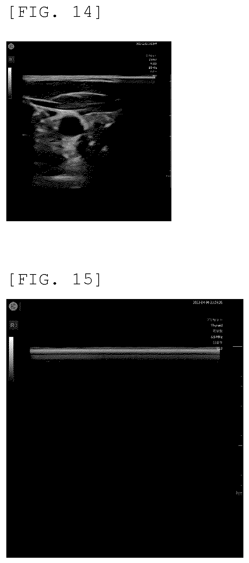

FIGs. 14 and 15 are the measured ultrasonograms, in which FIG. 14 shows a round cross section of the carotid artery, which is determined as good, and FIG. 15 shows no image, which is determined as poor.

### <Method for Forming Layered Structure>

In the present invention, an addition-curable silicone coupling material composition containing a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst can be applied on a release liner film. The applied addition-curable silicone coupling material composition can be heated and cured to form a silicone coupling material film. Then, the silicone coupling material film can be affixed on a gel coupling material film made of a gel containing water. An ultrasound probe can be attached on a surface of the gel coupling material film opposite a surface in contact with the silicone coupling material film. Thus, a layered structure can be formed.

Further, a layered structure can be also formed by affixing the gel coupling material film to the ultrasound probe through stripping the silicone coupling material film from the gel coupling material film.

In this way, by forming the layered structure appropriately, ultrasonic inspection can be performed efficiently.

### EXAMPLE

The present invention will be specifically described below using Examples and Comparative Examples. However, the present invention is not limited thereto.

### Examples 1 and 6, Comparative Examples 1 and 2

### (Component A)

A vinyl group-containing polydimethylsiloxane with a viscosity of 27,000 mPa·s in 49% toluene solution, an alkenyl group content of 0.02 mol/100 g, and a molecular chain end capped with a SiMe₂Vi group was used as a siloxane compound 1.

### (Component B)

KF-96 available from Shin-Etsu Chemical Co., Ltd. in Table 1 was used as a non-cross-linkable silicone oil. The kinematic viscosity at 25°C corresponds to the number immediately before CS in the table. For example, the kinematic viscosity of "KF-96 1,000CS" is 1,000 mm²/s and the kinematic viscosity of "KF-96 100CS" is 100 mm²/s. The kinematic viscosity is a value measured with an Ostwald viscometer at 25°C.

### (Component C)

KF-99 available from Shin-Etsu Chemical Co., Ltd. was used as a methylhydrogensilicone oil.

### (Additive)

### Dry silica: AEROSIL RX200 (available from Nippon Aerosil Co., Ltd., surface treated with a trimethylsilyl group)

A silicone ultrasonic coupling material solution was prepared according to the formulation listed in Table 1, and to 100 parts by mass of this solution, 1 part by mass of a platinum catalyst PL-56 from Shin-Etsu Chemical Co., Ltd. was added and further mixed.

**[Table 1]**

| Silicone ultrasonic coupling material solution | Component A (parts by mass) | Component B (parts by mass) | Component C (parts by mass) | B/ (A+B+C) (%) | SiH/Vi | Additive (parts by mass) |
|---|---|---|---|---|---|---|
| Silicone ultrasonic coupling material solution 1 | Siloxane compound 1 (100) | KF-96 1,000CS (140) | KF-99 (9.4) | 74.0 | 8.0 | AEROSIL RX200 (1.5) |
| Silicone ultrasonic coupling material solution 2 | Siloxane compound 1 (100) | KF-96 100CS (140) | KF-99 (10) | 74.0 | 8.3 | AEROSIL RX200 (1.5) |
| Silicone ultrasonic coupling material solution 3 | Siloxane compound 1 (100) | KF-96 50CS (140) | KF-99 (10) | 74.0 | 8.3 | AEROSIL RX200 (1.5) |
| Silicone ultrasonic coupling material solution 4 | Siloxane compound 1 (100) | KF-96 20CS (140) | KF-99 (10) | 74.0 | 8.3 | AEROSIL RX200 (1.5) |
| Silicone ultrasonic coupling material solution 5 | Siloxane compound 1 (100) | KF-96 100CS (175) | KF-99 (10) | 78.0 | 8.3 | AEROSIL RX200 (2.0) |
| Silicone ultrasonic coupling material solution 6 | Siloxane compound 1 (100) | KF-96 100CS (200) | KF-99 (10) | 80.2 | 8.3 | AEROSIL RX200 (2.5) |
| Comparative silicone ultrasonic coupling material solution 1 | Siloxane compound 1 (100) | - | KF-99 (10) | 0 | 8.3 | AEROSIL RX200 (2.5) |

### (Formation of Silicone Ultrasonic Coupling Material Film)

The silicone ultrasonic coupling material solution was applied on a release liner PET separator SS1A (50 µm thick) available from NIPPA CORPORATION with a bar coater, and after air drying for 10 minutes, the solution was baked in a hot air oven at 125°C for 10 minutes for curing, resulting in a coupling film illustrated in FIG. 1. After curing, a release liner PET separator SS1A (50 µm thick) available from NIPPA CORPORATION was placed thereon to produce a silicone ultrasonic coupling film sandwiched between two release liners.

### (Measurement of Thickness of Body Contact Layer)

The thickness of the ultrasonic coupling film produced above was measured using a micrometer. Table 2 shows the result.

### (Formation of Adhesive Water-Gel Coupling Material Film)

An acrylic adhesive with a thickness of about 50 µm was applied on both sides of a water-containing urethane gel Yasojima Echo Gel Pad EP-S-03 (3 mm thick) available from Yasojima Proceed Co., Ltd. to produce an adhesive water-gel coupling material film.

By removing the release liner on one side, the silicone ultrasonic coupling material film was affixed to the adhesive gel coupling material film to produce a composite ultrasonic coupling material film (FIG. 2).

### (Ultrasound Signal Measurement)

SONON 500L from Healcerion Co., Ltd. was used as a hand-held ultrasound diagnostic device (diagnostic instrument) for measuring ultrasound signals. The adhesive water-gel ultrasonic coupling material film side of the composite ultrasonic coupling material film was affixed to the probe surface of the ultrasound diagnostic device, and the release liner on the silicone ultrasonic coupling material was removed (FIG. 3).

The layered ultrasonic coupling material film with the silicone ultrasonic coupling material film was repeatedly affixed to and uncoupled from the skin 10 times to confirm smooth uncoupling from the skin and no separation between the silicone ultrasonic coupling layer and the adhesive water-gel ultrasonic coupling material film (FIG. 4).

After the position of a carotid artery was identified by attaching and uncoupling 10 times, the coupling material film was attached to the skin to perform ultrasonic diagnosis at a frequency of 8.5 MHz.

After smooth uncoupling of the silicone ultrasound coupling material film 1 was confirmed, the adhesive water-gel ultrasonic coupling material film was affixed to the skin to perform ultrasonic diagnosis at a frequency of 8.5 MHz (FIG. 5).

A case in which the image in FIG. 14 developed was determined as good, and a case in which the image in FIG. 15 developed was determined as poor.

Table 2 shows the evaluation results of composite ultrasonic coupling material films using the silicone ultrasonic coupling materials listed in Table 1.

**[Table 2]**

| Ex. | Silicone ultrasonic coupling material solution | Silicone ultrasonic coupling material thickness (micron) | Application and uncoupling on skin | Layered ultrasonic coupling material film ultrasonogram | Ultrasonogram after stripping silicone ultrasonic coupling material film |
|---|---|---|---|---|---|
| Ex.1 | Silicone ultrasonic coupling material solution 1 | 330 | Good | Good | Good |
| Ex.2 | Silicone ultrasonic coupling material solution 2 | 340 | Good | Good | Good |
| Ex.3 | Silicone ultrasonic coupling material solution 3 | 310 | Good | Good | Good |
| Ex. 4 | Silicone ultrasonic coupling material solution 4 | 296 | Good | Good | Good |
| Ex.5 | Silicone ultrasonic coupling material solution 5 | 321 | Good | Good | Good |
| Ex.6 | Silicone ultrasonic coupling material solution 6 | 302 | Good | Good | Good |
| Comp.Ex.1 | Comparative silicone coupling material solution 1 | 330 | Good | Poor | Good |
| Comp.Ex.2 | - | - | Poor | - | Good |

As shown in Table 2, in a case where attached to the skin was the composite film which includes the silicone ultrasonic coupling material film in Examples with the silicone oil of the component (B) added to the silicone rubber of the component (A) and the adhesive water-gel ultrasonic coupling material film, attaching and uncoupling on the skin can be performed many times, and a good ultrasonogram can be obtained. In addition, the silicone ultrasonic coupling material film can be easily stripped off, after stripped the silicone ultrasonic coupling material film, the adhesive water-gel ultrasonic coupling material film can be affixed to the skin, and a good ultrasonogram can be obtained. In a case of the composite film which includes the silicone ultrasonic coupling material film with no silicone oil of the component (B) added and the adhesive water-gel ultrasonic coupling material, an ultrasonogram could not be obtained (Comparative Example 1). In a case of the adhesive water-soluble gel coupling material alone, a good ultrasonogram was obtained, but application and uncoupling on the skin were failed (Comparison Example 2).

The present description includes the following embodiments.
[1]: An ultrasonic coupling material composite film to be inserted between an ultrasound probe surface and a skin, the ultrasonic coupling material composite film comprising: a gel coupling material film comprising a gel containing water; and a silicone coupling material film comprising a cross-linkable silicone resin and a silicone oil having no cross-linking point.
[2]: The ultrasonic coupling material composite film of the above [1], wherein the gel coupling material film has adhesiveness.
[3]: The ultrasonic coupling material composite film of the above [1] or [2], wherein the silicone coupling material film comprises a cured product of an addition-curable silicone coupling material composition comprising a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst.
[4]: The ultrasonic coupling material composite film of the above [3], wherein a number of moles of SiH groups of the component (C) divided by a number of moles of alkenyl groups of the component (A) is in a range of 0.5 to 20.
[5]: The ultrasonic coupling material composite film of the above [3] or [4], wherein the component (B) is a linear or branched diorganopolysiloxane which has no a cross-linking point, does not have an alkenyl group and a SiH group, and has a kinematic viscosity in a range of 10 to 50000 mm²/s at 25°C.
[6]: The ultrasonic coupling material composite film of any one of the above [3] to [5], wherein a mass of the component (B) divided by a total mass of the component (A), the component (B), and the component (C) is in a range of 0.20 to 0.90.
[7]: The ultrasonic coupling material composite film of any one of the above [3] to [6], wherein the addition-curable silicone coupling material composition further comprises 0.1 to 10 parts by mass of dry silica per 100 parts by mass of a total of the components (A) to (C).
[8]: The ultrasonic coupling material composite film of any one of the above [1] to [7], wherein the silicone coupling material film has a thickness in a range of 10 to 5000 µm.
[9]: The ultrasonic coupling material composite film of any one of the above [1] to [8], wherein the ultrasonic coupling material composite film further comprises membranes on both sides of the gel coupling material film and adhesive films outside the membranes.
[10]: The ultrasonic coupling material composite film of any one of the above [1] to [8], wherein the ultrasonic coupling material composite film further comprises a release liner film on one side or both sides of the ultrasonic coupling material composite film.
[11]: An ultrasonic inspection method using the ultrasonic coupling material composite film of any one of the above [1] to [9], excluding methods for operating on, treating, or diagnosing humans, the method comprising:
   inserting the ultrasonic coupling material composite film between an ultrasound probe surface and a subject such that the gel coupling material film of the composite film faces the ultrasound probe surface and the silicone coupling material film of the composite film faces the subject; and
   bringing the silicone coupling material film into contact with the subject to search for an ultrasonic inspection point.
[12]: The ultrasonic inspection method of the above [11], wherein the ultrasonic inspection point is searched for by repeating contact with the subject and uncoupling from the subject or by sliding and moving on the subject.
[13]: The ultrasonic inspection method of the above [11] or [12], wherein continuous ultrasonic inspection is performed by uncoupling the silicone coupling material film and then immobilizing the gel coupling material film in contact with the subject.
[14]: A method for forming a layered structure, the method comprising: applying, on a release liner film, an addition-curable silicone coupling material composition comprising a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst, and heating and curing the addition-curable silicone coupling material composition to form a silicone coupling material film; then affixing the silicone coupling material film on a gel coupling material film comprising a gel containing water; and attaching an ultrasound probe on a surface of the gel coupling material film opposite a surface in contact with the silicone coupling material film to form a layered structure.
[15]: The method for forming a layered structure of the above [14], wherein the gel coupling material film is affixed to the ultrasound probe by stripping the silicone coupling material film from the gel coupling material film.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. An ultrasonic coupling material composite film to be inserted between an ultrasound probe surface and a skin, the ultrasonic coupling material composite film comprising: a gel coupling material film comprising a gel containing water; and a silicone coupling material film comprising a cross-linkable silicone resin and a silicone oil having no cross-linking point.

2. The ultrasonic coupling material composite film according to claim 1, wherein the gel coupling material film has adhesiveness.

3. The ultrasonic coupling material composite film according to claim 1 or 2, wherein the silicone coupling material film comprises a cured product of an addition-curable silicone coupling material composition comprising a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst.

4. The ultrasonic coupling material composite film according to claim 3, wherein a number of moles of SiH groups of the component (C) divided by a number of moles of alkenyl groups of the component (A) is in a range of 0.5 to 20.

5. The ultrasonic coupling material composite film according to claim 3 or 4, wherein the component (B) is a linear or branched diorganopolysiloxane which has no a cross-linking point, does not have an alkenyl group and a SiH group, and has a kinematic viscosity in a range of 10 to 50000 mm²/s at 25°C.

6. The ultrasonic coupling material composite film according to any one of claims 3 to 5, wherein a mass of the component (B) divided by a total mass of the component (A), the component (B), and the component (C) is in a range of 0.20 to 0.90.

7. The ultrasonic coupling material composite film according to any one of claims 3 to 6, wherein the addition-curable silicone coupling material composition further comprises 0.1 to 10 parts by mass of dry silica per 100 parts by mass of a total of the components (A) to (C).

8. The ultrasonic coupling material composite film according to any one of claims 1 to 7, wherein the silicone coupling material film has a thickness in a range of 10 to 5000 µm.

9. The ultrasonic coupling material composite film according to any one of claims 1 to 8, wherein the ultrasonic coupling material composite film further comprises membranes on both sides of the gel coupling material film and adhesive films outside the membranes.

10. The ultrasonic coupling material composite film according to any one of claims 1 to 8, wherein the ultrasonic coupling material composite film further comprises a release liner film on one side or both sides of the ultrasonic coupling material composite film.

11. An ultrasonic inspection method using the ultrasonic coupling material composite film according to any one of claims 1 to 9, excluding methods for operating on, treating, or diagnosing humans, the method comprising:
inserting the ultrasonic coupling material composite film between an ultrasound probe surface and a subject such that the gel coupling material film of the composite film faces the ultrasound probe surface and the silicone coupling material film of the composite film faces the subject; and
bringing the silicone coupling material film into contact with the subject to search for an ultrasonic inspection point.

12. The ultrasonic inspection method according to claim 11, wherein the ultrasonic inspection point is searched for by repeating contact with the subject and uncoupling from the subject or by sliding and moving on the subject.

13. The ultrasonic inspection method according to claim 11 or 12, wherein continuous ultrasonic inspection is performed by uncoupling the silicone coupling material film and then immobilizing the gel coupling material film in contact with the subject.

14. A method for forming a layered structure, the method comprising: applying, on a release liner film, an addition-curable silicone coupling material composition comprising a component (A) which is diorganopolysiloxane having an alkenyl group, a component (B) which is silicone oil having no cross-linking point, a component (C) which is organohydrogenpolysiloxane having a SiH group, and a component (D) which is platinum group metal catalyst, and heating and curing the addition-curable silicone coupling material composition to form a silicone coupling material film; then affixing the silicone coupling material film on a gel coupling material film comprising a gel containing water; and attaching an ultrasound probe on a surface of the gel coupling material film opposite a surface in contact with the silicone coupling material film to form a layered structure.

15. The method for forming a layered structure according to claim 14, wherein the gel coupling material film is affixed to the ultrasound probe by stripping the silicone coupling material film from the gel coupling material film.
